# EUROPEAN PATENT APPLICATION

(11) **EP 3 714 888 A1**
(43) Date of publication of application: **30.09.2020**
(21) Application number: 19305366.7
(22) Date of filing: 25.03.2019
(51) Int. Cl.: A61K 31/522, A23L 33/105, A61P 25/14

(54) **COMPOUNDS FOR USE IN THE TREATMENT OF ADCY5-RELATED DYSKINESIA**

(71) Applicant: ASSISTANCE PUBLIQUE - HÔPITAUX DE PARIS, 75004 Paris (FR)
(72) Inventor: MENERET, Aurélie, 75007 PARIS (FR); FLAMAND-ROZE, Emmanuel, 75012 PARIS (FR); MARIANI, Louise-Laure, 94160 SAINT MANDE (FR)
(74) Representative: Gevers & Orès

(57) **Abstract**

The present invention relates to compounds of formula (I), preferably caffeine, and compositions comprising said compounds, for use in the treatment of *ADCY5*-related dyskinesia.

## Description

The present invention relates to compounds and compositions comprising said compounds, for use in the treatment of ADCY5-related dyskinesia.

Paroxysmal dyskinesia (PD) is a rare group of movement disorders manifesting as abnormal involuntary movements that recur episodically and last only a brief time. They can be isolated or part of a more complex movement disorder. In particular, *ADCY5-*related dyskinesia is a childhood-onset hyperkinetic movement disorder due to mutations in the *ADCY5* gene, coding for adenylate cyclase type 5. Recurrent mutations suggest particular functional importance of residues 418 and 726 of the adenylate cyclase type 5 in disease pathogenesis (Chen et al., ADCY5-related dyskinesia: Broader spectrum and genotype-phenotype correlations. Neurology. 2015;85(23):2026-35.). *ADCY5-related* dyskinesia is characterized by pleiotropic paroxysmal dyskinesia, with diurnal and nocturnal episodes, and a permanent hyperkinetic movement disorder with marked fluctuations of the motor state (Friedman et al., ADCY5 mutation carriers display pleiotropic paroxysmal day and night time dyskinesias. Mov Disord. 2016;31(1):147-8). Symptoms include abnormal movements affecting the limbs, trunk, face and/or neck. In mild cases, the abnormal movements minimally affect function but can be socially debilitating, whereas more severe cases may affect the ability to ambulate, leading to confinement in a wheelchair. Currently no effective treatment exists.

There thus remains a genuine need for an effective treatment of *ADCY5*-related dyskinesia.

The present invention is believed to meet such need by providing compounds and compositions for treating *ADCY5*-related dyskinesia.

Caffeine is a natural stimulant of the central nervous system. It is most commonly found in coffee, tea and cocoa plants and derived alimentary products. Unlike many other psychoactive substances, it is legal and unregulated in nearly all parts of the world.

Surprisingly, the Inventors have demonstrated that caffeine intake induces a near-complete resolution of episodes of dyskinesia in a patient suffering from paroxysmal dyskinesia. Until then, caffeine was rather known as a triggering factor for paroxysmal non-kinesigenic dyskinesia due to *PNKD* mutations (Méneret & Roze, Paroxysmal movement disorders: An update. Revue Neurol. 2016; 172(8-9):433-445).

The major targets of caffeine are adenosine receptors type 2A (A_{2A} R), which are promising therapeutic targets for a wide range of conditions including ischemic disease, sleep disorders, inflammatory disorders and cancer. Interestingly, A_{2A} receptors also couple to adenylate cyclase type 5 in the striatum (Xie et al., Adenosine and dopamine receptor interactions in striatum and caffeine-induced behavioral activation. Comp Med. 2007;57 (6):538-45).

In this context, the present invention concerns a compound of formula (I): 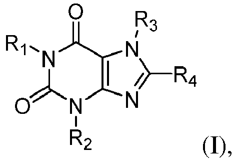 wherein :
R₁ is selected from H, CH₃ and C₂H₅,
R₂ is selected from H, CH₃ and C₂H₅,
R₃ is selected from H, CH₃ and C₂H₅,
R₄ is selected from H, CH₃, C₂H₅ and 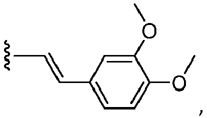
or pharmaceutically acceptable salts thereof,
for use in the treatment of *ADCY5*-related dyskinesia.

The present invention further relates to a composition comprising at least one compound as defined above as active ingredient and at least one pharmaceutically acceptable excipient, for use in the treatment of *ADCY5*-related dyskinesia.

The present invention further relates to a method for treating *ADCY5*-related dyskinesia, by means of administration, to a patient in need thereof, of an effective amount of a compound as defined above.

The present invention further relates to the use of a compound as defined above for the manufacture of a medication for treating *ADCY5*-related dyskinesia.

In some embodiments, the compound according to the invention is selected from the group consisting of: 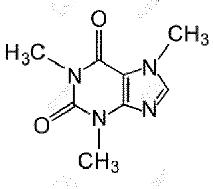 (1,3,7-trimethylxanthine, caffeine), 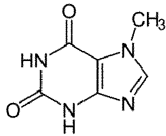 (7-methylxanthine), 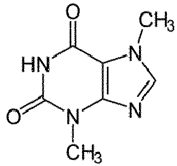 (3,7-dimethylxanthine, theobromine), 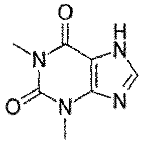 (1,3-dimethylxanthine, theophylline), 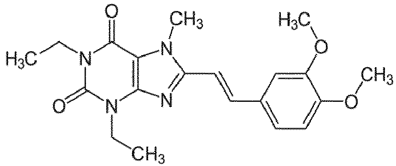 (istradefylline),
and pharmaceutically acceptable salts thereof.

In preferred embodiments, the compound of the invention is caffeine or pharmaceutically acceptable salt thereof.

In the present invention, the term "caffeine", also known as "theine", "guaranine" or "methyltheobromine", refers to the molecule 1,3,7-trimethylxanthine.

Caffeine is naturally present in some plants, in particular in coffee, tea, cocoa, guarana, kola and yerba mate. Caffeine can be extracted from such plants, in particular from beans, nuts, leaves or fruits, by various processes, which are well known by one skilled in the art and the industry.

In some embodiments, the compound according to the invention is a pharmaceutically acceptable salt of caffeine, preferably caffeine citrate.

In some embodiments, the compound according to the invention is an antagonist of the A_{2A} receptor, preferably a selective antagonist of the A_{2A} receptor. The selective antagonism to the A_{2A} receptor can be demonstrated via binding assays or using rodent models knockout for specific adenosine receptor subtypes.

In some embodiments, the compound according to the invention corresponds to a compound naturally produced during synthesis of caffeine by plants or during degradation of caffeine by living organisms (mammals, humans, bacteria, fungi, yeast, ...), such as 7-methylxanthine, 3,7-dimethylxanthine (theobromine) or 1,3-dimethylxanthine (theophylline).

By "pharmaceutically acceptable excipient" is meant, according to the invention, a non-pharmaceutically active additive used in the manufacture of a pharmaceutical composition, which allows the pharmaceutically active ingredient to be manufactured into a pharmaceutical formulation or a galenic formulation providing the necessary bioavailability of the medicament to the patient upon the administration of the pharmaceutical composition. The excipient is preferably compatible with the other ingredients of the composition and produces no adverse effect, allergic reaction or other undesirable reaction when it is administered to a human or an animal.

In the present invention, the term "paroxysmal dyskinesia" refers to abnormal involuntary movements that recur episodically. Paroxysmal dyskinesia can be triggered by voluntary movements in the case of paroxysmal kinesigenic dyskinesia (PKD), not triggered by voluntary movements in the case of paroxysmal non-kinesigenic dyskinesia (PNKD) or triggered by physical exercise in the case of paroxysmal exercise-induced dyskinesia (PED). All three subtypes may co-exist in *ADCY5*-related dyskinesia, as well as an interictal movement disorder of variable severity.

In the present invention, the term "*ADCY5*-related dyskinesia", formerly known as "familial dyskinesia with facial myokymia" or "FDFM", designates dyskinesia caused by mutations in the *ADCY5* gene. The diagnosis of *ADCY5*-related dyskinesia can be established with genetic testing to detect a pathogenic variant in *ADCY5.* Genetic testing methods may include sequence analysis of *ADCY5* or use of a multi-gene panel that includes *ADCY5* and other genes of interest. This condition is inherited in an autosomal dominant pattern, which means that one copy of the altered gene is sufficient to cause the disorder. In some cases, an affected person inherits the mutation from one affected parent. Other cases result from new mutations in the gene and occur in people with no history of the disorder in their family. Some of the abnormal movements that occur in *ADCY5*-related dyskinesia are permanent, whereas others appear as sudden (paroxysmal) jerks, twitches, tremors, muscle tensing (dystonia), or writhing (choreiform) movements, and can affect the limbs, neck, trunk and face.

By "treatment" is meant, according to the present invention, the decrease or the disappearance of permanent and paroxysmal dyskinesia. In other words, the treatment corresponds to a reduction, preferably a suppression, of the abnormal involuntary movements in the patients suffering from *ADCY5*-related dyskinesia.

The treatment according to the invention applies to humans or animals.

The compound or the composition according to the invention can be administered by enteral route, in particular by oral or rectal administration, or by parenteral route, in particular by injection.

The compound or the composition according to the invention may be in any forms allowing its administration by patient, for example a beverage, a food, a pill, a tablet, a syrup, a patch, a gum, a powder, a capsule, a vial, etc ...

In preferred embodiments, the compound or the composition according to the invention is in the form of a beverage or a food product.

In more preferred embodiments, the compound or the composition according to the invention is in the form of a beverage or a food, comprising or consisting of coffee beans, tea leaves, cocoa nuts, kola nuts, guarana seeds and yerba mate leaves.

In an embodiment, the compound or the composition according to the invention is formulated into unit dose forms from 10 to 1 000 mg, preferably from 50 to 500 mg, more preferably from 100 to 300 mg of said compound.

In an embodiment, the compound or the composition according to the invention is formulated into unit dose forms of 10, 20, 30, 40, 50, 60, 70, 80, 90, 100, 150, 200, 250, 300, 350, 400, 450, 500, 550, 600, 650, 700, 750, 800, 850, 900, 950 or 1 000 mg of said compound.

The compound or the composition according to the invention can be administered one or more times per day. The regimen can be easily adjusted by the person skilled in the art or the practitioner.

In an embodiment, the compound or the composition according to the invention is administered once daily, twice daily, three times daily or four times daily.

In an embodiment, the compound or the composition according to the invention is administered three times daily with an uptake of 100 mg of said compound in the morning, an uptake of 100 mg of said compound in the afternoon and an uptake of 50 mg of said compound at bedtime.

In an embodiment, caffeine is administered three times daily with an uptake of 100 mg in the morning, an uptake of 100 mg in the afternoon and an uptake of 50 mg at bedtime, preferably in the form of a coffee drink, such as an expresso.

In an embodiment, the compound or the composition according to the invention is administered four times daily with an uptake of 100 mg of said compound in the morning, an uptake of 100 mg of said compound in the afternoon, an uptake of 100 mg of said compound at bedtime and an uptake of 100 mg of said compound at night.

In an embodiment, caffeine is administered four times daily with an uptake of 100 mg in the morning, an uptake of 100 mg in the afternoon, an uptake of 100 mg at bedtime and an uptake of 100 mg at night, preferably in the form of a coffee drink, such as an expresso.

In another aspect, the present invention also relates to any compound, any composition or any method as defined above for the treatment of subtypes of dystonia due to a defect in the D1 and D2 dopaminergic pathways in the striatum, such as dystonia due to mutations in the gene *GNAL* or *PDE10A*-related dyskinesia.

*GNAL* codes for Gα(olf), which couples dopamine type 1 receptors and adenosine A_{2A} receptors to the activation of adenylate cyclase type 5, and *PDE10A* encodes phosphodiesterase 10A, which degrades cAMP formed by adenylate cyclase type 5. Thus, the molecular pathways implicated in these disorders are comparable to the molecular pathway causing *ADCY5*-related dyskinesia.

Consequently, all the embodiments of compounds, compositions and methods described above for their use in the treatment of *ADCY5*-related dyskinesia apply *mutatis mutandis* to the treatment of subtypes of dystonia due to a defect in the D1 and D2 dopaminergic pathways in the striatum.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

### EXAMPLE 1

Two patients, a father and a daughter, were diagnosed by the Inventors as suffering from *ADCY5*-related dyskinesia. They had diurnal and nocturnal paroxysmal dyskinesia as well as a mild permanent movement disorder. They had self-medicated for years with coffee, which they said could prevent the occurrence of episodes. According to the father, he would never go to bed without drinking coffee, or else he would be woken up by dyskinesia.

The below experiments were carried out to report in detail the efficacy of caffeine in another patient with *ADCY5*-related dyskinesia and discuss the rationale supporting caffeine use in this condition.

The patient is an 11-year-old boy with the disorder. Pregnancy, birth and first acquisitions were unremarkable. At the age of three, he developed diurnal and nocturnal episodes of hyperkinetic involuntary movements involving the face and upper limbs, lasting from a few seconds to ten minutes. There was a clear kinesigenic trigger for some of the episodes, others were exercise-induced, and a few were spontaneous. In addition, he was described as clumsy, and had difficulties in various activities of daily living such as fine motor tasks or riding a bike. Frequency of the episodes was around 30 per day at age 11, causing significant disruption of activities. For example, he had trouble writing in class, was not able to walk home from school and could not do sports. Interictal neurological examination was normal except for some fleeting choreodystonic movements. Because of the co-occurrence of several types of paroxysmal dyskinesia, the patient was first tested for mutations in the *SLC2A1* and *ADCY5* genes. Genetic analysis was negative for *SLC2A1* but found a mosaic heterozygous mutation in the *ADCY5* gene (c.2088+1G>A), previously reported as pathogenic (Carapito et al., A de novo ADCY5 mutation causes early-onset autosomal dominant chorea and dystonia. Mov Disord. 2015;30(3):423-7).

Caffeine was the first treatment tried. The parents were not surprised by the coffee prescription, as it is a commonly used medicine for various ailments in both children and adults in Madagascar, their country of origin. He was first started on one espresso in the morning, approximately 100 mg of caffeine, with a dramatic response starting 45 minutes after intake and lasting for seven hours. He then took a second espresso in the afternoon and half an espresso at bedtime, with next to complete resolution of the diurnal and nocturnal episodes. There were at most one or two short and non-disruptive episodes during the day. The patient was able to resume regular activities such as writing in class or walking home from school, and even started riding a bike again.

In a fortuitous, real-life double-blind experiment, the parents unknowingly bought decaffeinated coffee, resulting in immediate reversal to baseline for four days until the mistake was discovered. Caffeine intake immediately restored the improvement noted before.

ADCY5-related dyskinesia is believed to be due to gain of function mutations of adenylate cyclase type 5, which is mainly expressed in the striatum. In the striato-pallidal neurons, adenylate cyclase type 5 is inhibited by dopamine through D2 receptors and activated by adenosine through A_{2A} receptors (A_{2A}R), whose density is high in the striatum. Antagonizing A_{2A}R and thereby inhibiting adenylate cyclase type 5 therefore makes sense in order to reduce the hyperkinetic movement disorder seen in patients with ADCY5-related dyskinesia. Caffeine is the most commonly consumed drug in the world and is an antagonist of adenosine receptors. It probably acts primarily through A_{2A}R, as its wake-promoting effect is abolished in A_{2A}R knockout mice (Huang et al., Adenosine A2A, but not A1, receptors mediate the arousal effect of caffeine. Nat Neurosci. 2005;8(7):858-9.). In light of this strong rationale and clinical experience of the Inventors, caffeine and analogous compounds antagonists of adenosine receptors 2A appear to be effective and promising treatments in patients with *ADCY5*-related dyskinesia.

### EXAMPLE 2

Currently, seven patients are treated with caffeine with similar results with uptakes varying from 150 to 800 mg of caffeine per day. In all cases, it is observed a reduction or a suppression of dyskinesia episodes. Four of them were asked to rate the improvement brought by caffeine on their involuntary movements, on a scale of 0 (no improvement) to 100% (major improvement with total disappearance of symptoms). All four reported an improvement of 80 to 100%.

## Claims

1. A compound of formula (I): 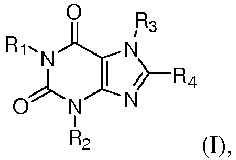 wherein :
R₁ is selected from H, CH₃ and C₂H₅,
R₂ is selected from H, CH₃ and C₂H₅,
R₃ is selected from H, CH₃ and C₂H₅,
R₄ is selected from H, CH₃, C₂H₅ and 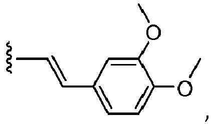
or pharmaceutically acceptable salts thereof,
for use in the treatment of ADCY5-related dyskinesia.

2. A compound for use according to claim 1, wherein said compound is selected from the group consisting of: 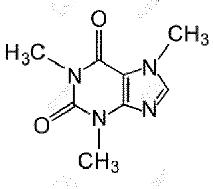 (1,3,7-trimethylxanthine, caffeine), 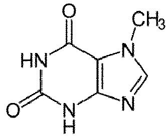 (7-methylxanthine), 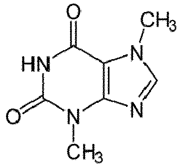 (3,7-dimethylxanthine, theobromine), 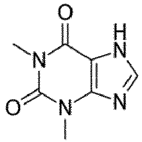 (1,3-dimethylxanthine, theophylline), 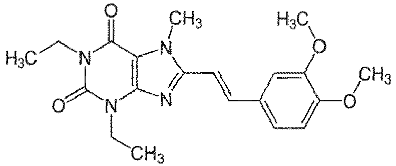 (istradefylline), and pharmaceutically acceptable salts thereof.

3. A compound for use according to claim 1 or 2, wherein said compound is caffeine or a pharmaceutically acceptable salt thereof.

4. A composition comprising at least one compound as defined in any one of claims 1 to 3 as active ingredient, and at least one pharmaceutically acceptable excipient, for use in the treatment of ADCY5-related dyskinesia.

5. The compound or the composition for use according to any one of claims 1 to 4, wherein said compound or said composition is administered by enteral route, in particular by oral or rectal administration, or by parenteral route, in particular by injection.

6. The compound or the composition for use according to any one of claims 1 to 5, wherein said compound or said composition is in the form of a beverage, a food, a pill, a tablet, a syrup, a patch, a gum, a powder, a capsule or a vial.

7. The compound or the composition for use according to any one of claims 1 to 6, wherein said compound or said composition is in the form of a beverage or a food comprising or consisting of coffee beans, tea leaves, cocoa nuts, kola nuts, guarana seeds and yerba mate leaves.

8. The compound or the composition for use according to any one of claims 1 to 7, wherein said compound or said composition is formulated into unit dose forms from 10 to 1 000 mg, preferably from 50 to 500 mg, more preferably from 100 to 300 mg of said compound.

9. The compound or the composition for use according to any one of claims 1 to 8, wherein said compound or said composition is formulated into unit dose forms of 10, 20, 30, 40, 50, 60, 70, 80, 90, 100, 150, 200, 250, 300, 350, 400, 450, 500, 550, 600, 650, 700, 750, 800, 850, 900, 950 or 1 000 mg of said compound.

10. The compound or the composition for use according to any one of claims 1 to 9, wherein said compound or said composition is administered one or more times per day.

11. The compound or the composition for use according to any one of claims 1 to 10, wherein said compound or said composition is administered once daily, twice daily, three times daily or four times daily.

12. The compound or the composition for use according to any one of claims 1 to 11, wherein caffeine or a composition comprising caffeine is administered three times daily with an uptake of 100 mg of caffeine in the morning, an uptake of 100 mg in the afternoon and an uptake of 50 mg at bedtime, preferably in the form of a coffee drink.

13. The compound or the composition for use according to any one of claims 1 to 11, wherein caffeine or a composition comprising caffeine is administered four times daily with an uptake of 100 mg of caffeine in the morning, an uptake of 100 mg in the afternoon, an uptake of 100 mg at bedtime and an uptake of 100 mg at night, preferably in the form of a coffee drink.
